(19) Europäisches Patentamt
European Patent Office
Office européen des brevets

(11) Publication number: **0 388 619 B1**

# (12) EUROPEAN PATENT SPECIFICATION

(45) Date of publication of patent specification: **10.08.94**

(51) Int. Cl.5: **C07D 213/807**

(21) Application number: **90102551.0**

(22) Date of filing: **09.02.90**

The file contains technical information submitted after the application was filed and not included in this specification

## (54) Method for the sequential oxidation of substituted quinolines to produce substituted-pyridine-2,3-dicarboxylic acids.

(30) Priority: **22.03.89 US 326940**

(43) Date of publication of application:
**26.09.90 Bulletin 90/39**

(45) Publication of the grant of the patent:
**10.08.94 Bulletin 94/32**

(84) Designated Contracting States:
**AT BE CH DE DK ES FR GB GR IT LI LU NL SE**

(56) References cited:
**EP-A- 0 126 893**
**EP-A- 0 135 328**
**EP-A- 0 149 857**
**EP-A- 0 232 118**
**EP-A- 0 259 687**
**EP-A- 0 331 899**

(73) Proprietor: **AMERICAN CYANAMID COMPANY**
**One Cyanamid Plaza**
**Wayne, NJ 07470-8426(US)**

(72) Inventor: **Pascavage, John Joseph**
**804 West 15th Street**
**Tyrone, Pennsylvania 16686(US)**

(74) Representative: **Wächtershäuser, Günter, Prof. Dr.**
**Patentanwalt**
**Tal 29**
**D-80331 München (DE)**

**EP 0 388 619 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention herein described relates to a method for the preparation of substituted pyridine-2,3-dicarboxylic acids via a sequential two part oxidation of the appropriately substituted quinoline precursors under basic conditions. Related subject matter can be found in United States Letters Patent Number 4,816,588 which describes a method for the preparation of substituted and unsubstituted pyridine-2,3-dicarboxylic acids under basic conditions and the advantages thereof over the prior art. The novel use of hydrogen peroxide in the presence of base to yield substituted-2,3-pyridine-dicarboxylic acids in high purity is a distinct advantage over the known methods for preparing said dicarboxylic acids.

It is an object of this invention to provide an improvement in the hydrogen peroxide-base oxidation process so as to produce high purity 2,3-pyridine-dicarboxylic acids in significantly increased yields via a sequential oxidation using hypochlorite (introduced as its alkalai metal salt or generated in situ) in the presence of aqueous base.

The European application EP-A-331899 discloses a method for the sequential oxidation of some substituted-8-hydroxy-quinolines. This application was published the 13/09/89, i.e. after the present priority date.

It has been found that the sequential addition of hypochlorite to the hydrogen peroxide-base oxidation surprisingly decreases the amounts of hydrogen peroxide needed for optimum product purity and significantly increases the product yield. The substituted pyridine-2,3-dicarboxylic acids, so produced, are useful as intermediates in the preparation of novel pyridine and quinoline imidazolinone herbicidal agents as described in United States Patent 4,518,780 and United States Patent 4,638,068. The appropriately substituted pyridine and quinoline 2,3-dicarboxylic anhydrides used as starting materials in said patents may be prepared according to the process described in United States Patent 4,562,257 from their pyridine and quinoline 2,3-dicarboxylic acid precursors. The sequence of reactions described in the above mentioned United States Patents to obtain useful herbicidal agents of formula IV from substituted pyridine 2,3-dicarboxylic acids of formula I is illustrated as Flow Diagram I.

FLOW DIAGRAM I

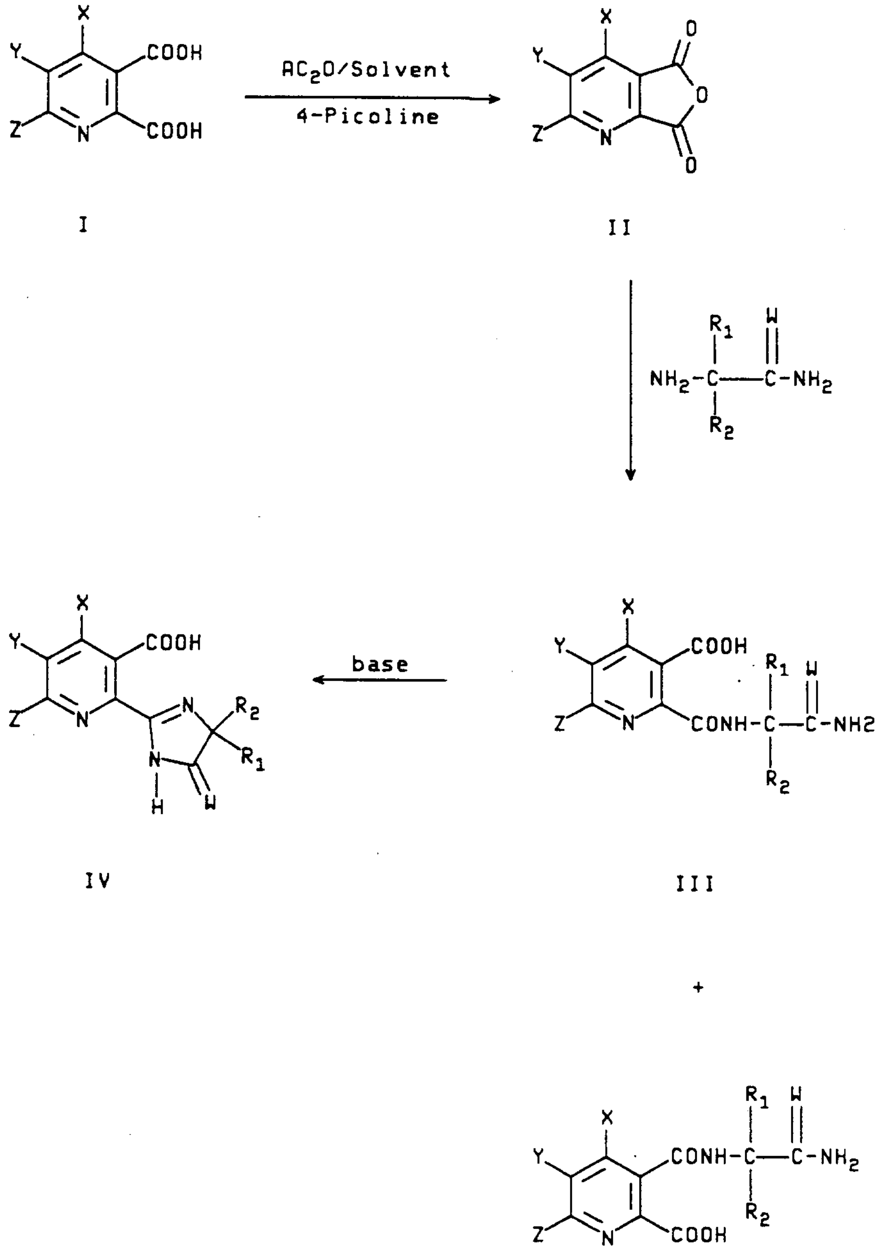


I

II

IV

III

The invention relates to a novel method as defined in claim 1 for the preparation of substituted pyridine-2,3-dicarboxylic acids of formula I

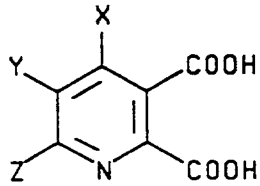

(I)

wherein

X is hydrogen, or methyl, with the proviso that when Y and Z are taken together to form a ring and YZ is represented by the structure: $-(CH_2)_n-$,where n is 3 or 4, X is hydrogen;

Y is hydrogen, halogen, $C_1$-$C_6$ alkyl, $C_1$-$C_6$ hydroxyalkyl, $C_1$-$C_6$ haloalkyl, $C_1$-$C_6$ aminoalkyl, $C_1$-$C_6$ sulfonylalkyl, nitro, hydroxy, formyl, carboxy, acyl, amido, amino, $C_1$-$C_4$ alkylamino, diloweralkylamino, $C_1$-$C_4$ alxylsulfonyl, sulfonamido, or phenyl optionally substituted with one $C_1$-$C_4$ alkyl group, $C_1$-$C_4$ alkylsulfonyl group, halogen, hydroxy, or trifluoromethyl group;

Z is hydrogen, $C_1$-$C_6$ alkyl, $C_1$-$C_6$ hydroxyalkyl, $C_1$-$C_6$ haloalkyl, $C_1$-$C_6$ aminoalkyl, $C_1$-$C_6$ sulfonylalkyl, nitro, hydroxy, formyl, carboxy, acyl, amido, amino, $C_1$-$C_4$ alkylamino, diloweralkylamino, $C_1$-$C_4$ alkylsulfonyl, sulfonamido, or phenyl optionally substituted with one $C_1$-$C_4$ alkyl group, $C_1$-$C_4$ alkylsulfonyl group, halogen, hydroxy, or trifluoromethyl group; and when taken together,

Y and Z may form a ring in which YZ are represented by the structure: $-(CH_2)_n-$, where n is an integer selected from 3 or 4, provided that X is hydrogen; or

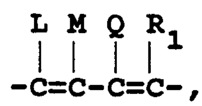

where L, M, Q, and $R_1$ each represent members selected from the group consisting of hydroxy, halogen, $C_1$-$C_4$ alkyl, $C_1$-$C_4$ alkylsulfonyl, $C_1$-$C_4$ haloalkylamino, $C_1$-$C_4$ alkylamino, diloweralkylamino, and trifluoromethyl, with the proviso that only one of L, M, Q or $R_1$ may represent a substituent other than hydrogen, halogen, or $C_1$-$C_4$ alkyl.

Compounds of formula I are prepared by oxidizing a substituted quinoline of formula II

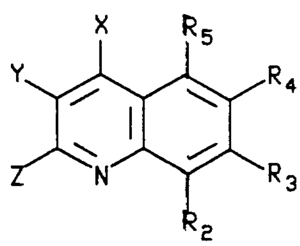

(II)

wherein

X, Y, and Z are as described for formula I above,

$R_2$, $R_3$, $R_4$ and $R_5$ are each hydrogen, hydroxy, $SO_3H$, $SO_2Cl$, SH, halogen, $NO_2$, $NH_2$; with the proviso that one of $R_2$, $R_3$, $R_4$ or $R_5$ is other than hydrogen and when $R_2$ is hydroxy, at least one of $R_3$, $R_4$ and $R_5$ is other than hydrogen;

the N-oxides thereof; the acid addition salts thereof; in the presence of aqueous base using hydrogen peroxide followed by addition of hypochlorite.

Aqueous bases suitable for use in the method of the invention are selected from alkali metal and alkaline earth metal hydroxides and carbonates such as sodium, potassium, lithium, and calcium hydroxides or carbonates and mixtures thereof. Aqueous sodium hydroxide and aqueous potassium hydroxide are the preferred bases.

In the presence of 2.0-10.0 molar equivalents of aqueous base (preferably 5.0-6.0 molar equivalents), quinolines of formula II are treated with about 7.0-20.0 molar equivalents of hydrogen peroxide, preferably 7.5-9.0 molar equivalents, at 25°-125°C, preferably 85°-90°C. Following the addition, the reaction temperature is maintained for at least one hour at 25°-125°C, preferably 85°-90°C. The reaction mixture is cooled to a temperature between about 25°-90°C, preferably about 65°-70°C and mineral acid is added to obtain a pH of about 8-14, preferably about 10.5-11.5. At this time, 1.0-4.0 molar equivalents (preferably 1.0-2.0 molar equivalents) of hypochlorite anion is added as a 5%-30% aqueous solution or is generated in situ by the direct addition of chlorine gas. Reaction temperatures of above 25°-125°C are suitable, however, additional reaction time is required at lower temperatures for complete oxidation to occur.

The oxidation of quinolines of formula II to pyridine-2,3-dicarboxylic acids of formula I according to the method of this invention is treated as a two part process. The initial step of the oxidation reaction is the cleavage of the nonhetero-aromatic ring bearing the functional group by hydrogen peroxide in the presence of aqueous base to give intermediates of formula IIa. The second part of the process is the oxidation of the side chains of formula IIa intermediates to carboxylic acid functional groups via the introduction of hypochlorite anions as illustrated in flow diagram II.

FLOW DIAGRAM II

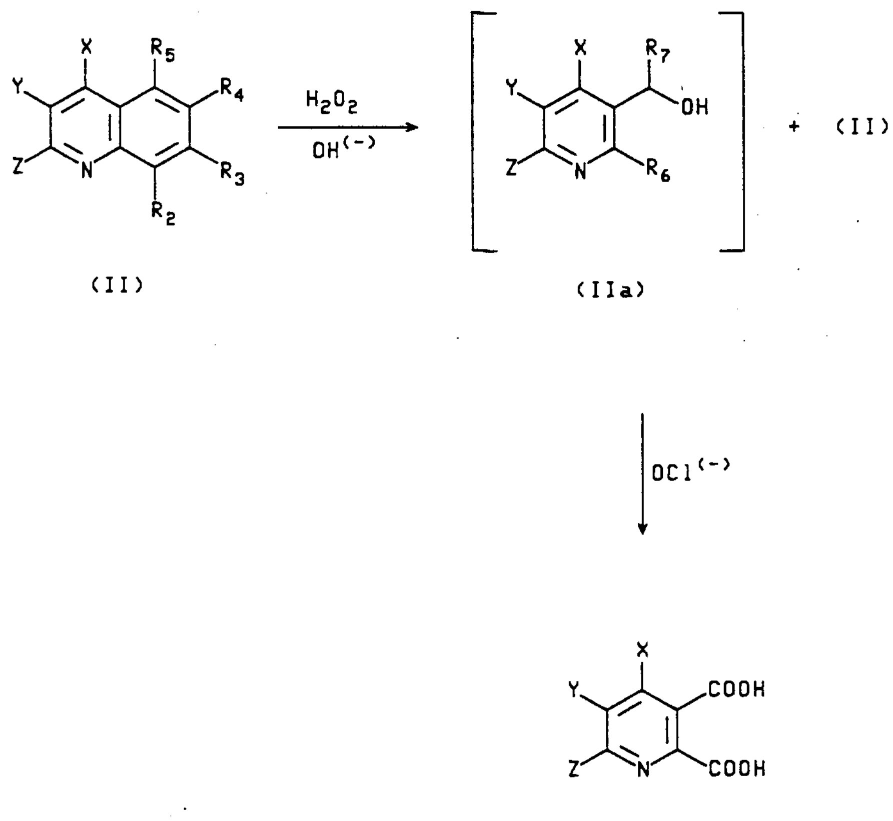


(II)

(IIa)

(I)

wherein $R_6$ and $R_7$ represent a mixture of members selected from the functional groups consisting of carboxylic acids, glycolic acids, aldehydes, hydroxymethyl groups, and other alkyl groups at intermediate stages of oxidation. It has been found that whereas hydrogen peroxide is preferable for the first oxidation step, i.e. cleavage of the aromatic ring system, surprisingly, hypochlorite anion is preferable for the completion of oxidation of the resulting intermediates to the final dicarboxylic acid products.

The pH of the reaction solution at the time of the introduction of the hypochlorite anion has a great influence on the reaction yield. It has been found that adjustment of the reaction pH to a range of about 10.5-11.5 gives an excellent yield of reaction product.

After the addition of hypochlorite as a 5%-30% aqueous solution or generated in situ by the addition of chlorine gas, the reaction is followed by using a potassium iodide-starch indicator test for the presence of

hypochlorite anions. When the potassium iodide-starch test is negative (usually after about one hour), the product dicarboxylic acid can be obtained by acidification of the reaction mixture with a mineral acid and isolated by standard procedures such as filtration or extraction into an appropriate organic solvent such as tetrahydrofuran, acetone, a $C_3$-$C_6$ alcohol, or a mono $C_1$-$C_4$ ether of ethylene glycol. A preferred organic solvent is tetrahydrofuran.

Among the compounds that can be prepared by this process are those shown below in Table I.

**Table I**

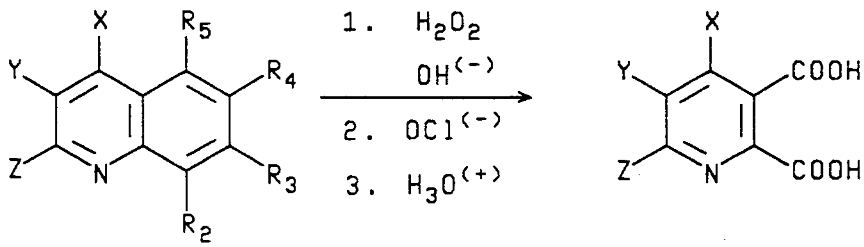


(II) (I)

| X | Y | Z |
|---|---|---|
| H | $CH_3$ | H |
| H | $C_2H_5$ | H |
| H | H | $CH_3$ |
| $CH_3$ | H | H |
| H | $C_2H_5$ | $C_2H_5$ |
| H | $H_2N$-$C_6$-$H_{12}$ | H |
| H | Br | H |
| H | $NH_2$ | H |
| H | $NH_2$ | $CH_3$ |
| H | $HOCH_2$ | H |
| H | $CH_2$=CH-$CH_2$ | $CH_3$ |
| H | $CH_2$=CH-$CH_2$ | H |
| H | -$SO_2NH_2$ | $CH_3$ |
| H | $CH_3$-CH(OH) | H |

Formula II quinolines which may be used in the herein-described process are: 3-ethyl-8-chloroquinoline, 8-chloroquinoline, 3-methyl-8-chloroquinoline, 5-hydroxyquinoline, 8-quinolinesulfonylchloride, 8-(3-ethylquinoline)sulfonic acid, 8-quinoline sulfonic acid, 5-nitro-8-hydroxyquinoline, and the like.

In order to facilitate a further understanding of the invention, the following examples are presented primarily for the purpose of illustrating more specific details thereof. Unless otherwise noted, all parts are by weight and all degrees are degree centigrade.

## EXAMPLE 1

### Preparation of pyridine-2,3-dicarboxylic acid via hydrogen peroxide and sodium hypochlorite

A stirred mixture of 8-aminoquinoline (7.21 g, 0.05 mole) and 15% aqueous potassium hydroxide (121.6 g, 0.325 mole) is treated with 8 molar equivalents of hydrogen peroxide as a 30% solution (45.3 g, 0.40 mole) at 80-90° over a one hour period. The reaction mixture is held at 80-90° for another 1/2 hour period, cooled to 65°, treated with 96% sulfuric acid to pH 11, and then treated with 15% sodium hypochlorite (44.7 g, 0.09 mole). The reaction temperature is maintained at a temperature between 65° and 70° for a total of 1 1/2 hours. The reaction mixture is cooled to 25° and the titled product is obtained in a 53.4% yield as determined by high pressure liquid chromatography analysis.

Using the above procedure, pyridine-2,3-dicarboxylic acid is prepared using quinolines of formula III as shown in Table I.

**Table I**

**Sequential oxidation of substituted quinolines to pyridine-2,3-dicarboxylic acid (PDC) via hydrogen peroxide and hypochlorite**

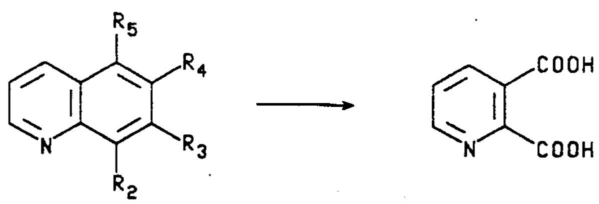

(III)

| Formula III Compound | | | | | Molar Equiv | % Yield |
|---|---|---|---|---|---|---|
| $R_2$ | $R_3$ | $R_4$ | $R_5$ | Base | Base | PDC |
| OH | H | H | Cl | KOH | 5.5 | 53.6 |
| $NO_2$ | H | H | H | KOH | 6.5 | 37.3 |
| H | H | H | OH | KOH | 5.5 | 58.5 |

## EXAMPLE 2

### Preparation of pyridine-2,3-dicarboxylic acid via the sequential oxidation of 8-quinolinesulfonyl chloride

A stirred mixture of 25 mL of water and 8-quinolinesulfonyl chloride (11.4 g, 0.05 mole) is treated with 85% potassium hydroxide (24.2 g, 0.375 mole) and heated to 250° while distilling off some water. The reaction mixture is held at 250° for 1/2 hour, cooled to 20°-50° and treated with 100 mL of water. The reaction mixture is then heated to 85° and treated with 8 molar equivalents of 30% hydrogen peroxide (45.3 g, 0.4 mole) over a one hour period at 85°-90°. After an additional 1/2 hour at 85° and cooling to 65°, 96% sulfuric acid is added to pH 11, followed by the addition of 1.8 molar equivalents of 15% sodium hypochlorite (44.7 g, 0.09 mole) over a 1/2 hour period at 65°-70°. The reaction mixture is held for an additional 1 hour at 65°-70°, cooled to room temperature to give the product pyridine-2,3-dicarboxylic acid, as determined by high pressure liquid chromatography (HPLC) analysis.

## Claims

**1.** A method for the preparation of pyridine-2,3-dicarboxylic acids of formula I

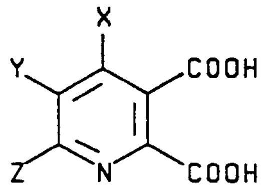

(I)

wherein

X is hydrogen, or methyl, with the proviso that when Y and Z are taken together to form a ring, and YZ is represented by the structure: $-(CH_2)_n-$, where n is 3 or 4, X is hydrogen;

Y is hydrogen, halogen, $C_1$-$C_6$ alkyl, $C_1$-$C_6$ hydroxyalkyl, $C_1$-$C_6$ haloalkyl, $C_1$-$C_6$ aminoalkyl, $C_1$-$C_6$ sulfonylalkyl, nitro, hydroxy, formyl, carboxy, acyl, amido, amino, $C_1$-$C_4$ alkylamino, diloweralkylamino, $C_1$-$C_4$ alkylsulfonyl, sulfonamido, or phenyl optionally substituted with one $C_1$-$C_4$ alkyl group, $C_1$-$C_4$ alkylsulfonyl group, halogen, hydroxy, or trifluoromethyl group;

Z is hydrogen, $C_1$-$C_6$ alkyl, $C_1$-$C_6$ hydroxyalkyl, $C_1$-$C_6$ haloalkyl, $C_1$-$C_6$ aminoalkyl, $C_1$-$C_6$ sulfonylalkyl, nitro, hydroxy, formyl, carboxy, acyl, amido, amino, $C_1$-$C_4$ alkylamino, diloweralkylamino, $C_1$-$C_4$ alkylsulfonyl, sulfonamido, or phenyl optionally substituted with one $C_1$-$C_4$ alkyl group, $C_1$-$C_4$ alkylsulfonyl group, halogen, hydroxy, or trifluoromethyl group; and when taken together,

Y and Z may form a ring in which YZ are represented by the structure: $-(CH_2)_n-$, where n is an integer selected from 3 or 4, provided that X is hydrogen; or

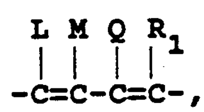

where L, M, Q, and $R_1$ each represent members selected from the group consisting of hydroxy, halogen, $C_1$-$C_4$ alkyl, $C_1$-$C_4$ alkylsulfonyl, $C_1$-$C_4$ haloalkylamino, $C_1$-$C_4$ alkylamino, diloweralkylamino, and trifluoromethyl, with the proviso that only one of L, M, Q or $R_1$ may represent a substituent other than hydrogen, halogen, or $C_1$-$C_4$ alkyl,

comprising reacting a quinoline compound of formula II

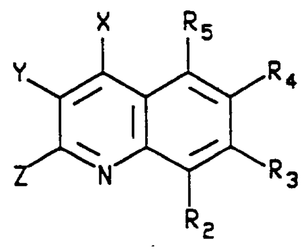

(II)

wherein

X, Y, and Z are as described for formula I above,

$R_2$, $R_3$, $R_4$ and $R_5$ are each hydrogen, hydroxy, $SO_3H$, $SO_2Cl$, SH, halogen, $NO_2$, $NH_2$; with the proviso that one of $R_2$, $R_3$, $R_4$ or $R_5$ is other than hydrogen and when $R_2$ is hydroxy at least one of $R_3$, $R_4$ and $R_5$ is other than hydrogen;

the N-oxides thereof; the acid addition salts thereof; with about 7.0-20.0 molar equivalents of hydrogen peroxide in the presence of 2.0-10.0 molar equivalents of aqueous base selected from the group consisting of alkali metal and alkaline earth metal hydroxides and carbonates and mixtures thereof, at a temperature range of about 25°C-125°C, cooling the thus-formed reaction mixture to a temperature below 125°C, treating the cooled reaction mixture with mineral acid to obtain a pH of about 8-14, treating the pH adjusted reaction mixture with 1.0-4.0 molar equivalents of hypochlorite and maintaining said reaction mixture at a temperature between about 25°C-125°C to yield the formula I pyridine-2,3-dicarboxylic acid.

**2.** A method according to claim 1 which further comprises isolating the formula I pyridine-2,3-dicarboxylic acid by acidification of said reaction mixture and extraction with an organic solvent.

**3.** A method according to claim 1 wherein about 7.5-9.0 molar equivalents of hydrogen peroxide and 5.0-6.0 molar equivalents of aqueous base are added to a stirred solution of a formula II quinoline.

**4.** A method according to claim 3 wherein the pH range of the reaction mixture is 10.5 to 11.5 at the time of the hypochlorite addition.

**5.** A method according to claim 4 wherein 1.0-2.0 molar equivalents of hypochlorite is generated in situ by the addition of chlorine gas.

**6.** A method according to claim 4 wherein 1.0-2.0 molar equivalents of hypochlorite is added as a 5%-15% aqueous solution.

**7.** A method according to claim 2 wherein the organic solvent is selected from a group consisting of tetrahydrofuran, acetone, $C_3$-$C_6$ alcohol, and mono $C_1$-$C_4$ ethers of ethylene glycol.

**8.** A method according to claim 7 wherein the organic solvent is tetrahydrofuran.

## Patentansprüche

**1.** Verfahren zur Herstellung von Pyridin-2,3-dicarbonsäuren der Formel I

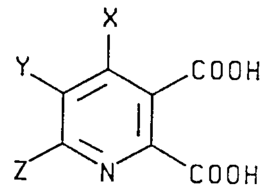

(I)

wobei

X für Wasserstoff oder Methyl steht, mit der Maßgabe, daß dann, wenn X und Y zusammen gefaßt sind, sie einen Ring bilden und YZ für die folgende Struktur steht: $-(CH_2)_n-$, wobei n für 3 oder 4 steht, X für Wasserstoff steht;

Y für Wasserstoff, Halogen, $C_{1-6}$-Alkyl, $C_{1-6}$-Hydroxyalkyl, $C_{1-6}$-Halogenalkyl, $C_{1-6}$-Aminoalkyl, $C_{1-6}$-Sulfonylalkyl, Nitro, Hydroxy, Formyl, Carboxy, Acyl, Amido, Amino, $C_{1-4}$-Alkylamino, Di-niederalkylamino, $C_{1-4}$-Alkylsulfonyl, Sulfonamido, oder Phenyl steht, das gegebenenfalls substituiert ist mit einer $C_{1-4}$-Alkylgruppe, $C_{1-4}$-Alkylsulfonylgruppe, Halogen, Hydroxy oder Trifluormethylgruppe;

Z für Wasserstoff, $C_{1-6}$-Alkyl, $C_{1-6}$-Hydroxyalkyl, $C_{1-6}$-Halogenalkyl, $C_{1-6}$-Aminoalkyl, $C_{1-6}$-Sulfonylalkyl, Nitro, Hydroxy, Formyl, Carboxy, Acyl, Amido, Amino, $C_{1-4}$-Alkylamino, Di-niederalkylamino, $C_{1-4}$-Alkylsulfonyl, Sulfonamido oder Phenyl steht, das gegebenenfalls substituiert ist mit einer $C_{1-4}$-Alkylgruppe, $C_{1-4}$-Alkylsulfonylgruppe, Halogen, Hydroxy oder Trifluormethylgruppe; und wobei dann, wenn sie zusammengefaßt sind, Y und Z einen Ring bilden können, in dem YZ für die Struktur: $-(CH_2)_n-$ steht, wobei n für eine ganze Zahl steht, ausgewählt aus 2 oder 4, mit der Maßgabe, daß X für Wasserstoff steht; oder für die Struktur

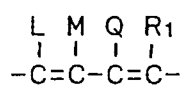

steht, wobei L, M, Q und $R_1$ jeweils Mitglieder repräsentieren, die ausgewählt sind aus der Gruppe, bestehend aus Hydroxy, Halogen, $C_{1-4}$-Alkyl, $C_{1-4}$-Alkyl-sulfonyl, $C_{1-4}$-Halogenalkyl, Amino, $C_{1-4}$-Alkylamino, Di-niederalkylamino und Trifluormethyl, mit der Maßgabe, daß nur einer von L, M, Q oder $R_1$ für einen Substituenten stehen kann, der nicht Wasserstoff, Halogen oder $C_{1-4}$-Alkyl ist, umfassend die Umsetzung einer Chinolinverbindung der Formel II

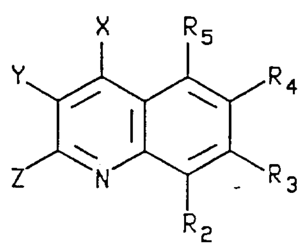

(II)

wobei

X, Y und Z wie oben für Formel I beschrieben sind,

$R_2$, $R_3$, $R_4$ und $R_5$ jeweils Wasserstoff, Hydroxy, $SO_3H$, $SO_2Cl$, SH, Halogen, $NO_2$, $NH_2$ bedeuten, mit der Maßgabe, daß eines von $R_2$, $R_3$, $R_4$ oder $R_5$ nicht Wasserstoff ist, und daß dann, wenn $R_2$ für Hydroxy steht, mindestens eines von $R_3$, $R_4$ und $R_5$ nicht Wasserstoff ist;

die N-Oxide derselben; die Säureadditionssalze derselben; mit etwa 7,0 bis 20,0 molaren Äquivalenten

Wasserstoffperoxid in Gegenwart von 2,0 bis 10,0 molaren Äquivalenten wässrige Base, ausgewählt aus der Gruppe, bestehend aus Alkalimetall- und Erdalkalimetallhydroxiden und -carbonaten und deren Mischungen bei einer Temperatur im Bereich von etwa 25 bis 125 °C, Abkühlung der so gebildeten Reaktionsmischung auf eine Temperatur unterhalb 125 °C, Behandlung der abgekühlten Reaktionsmischung mit Mineralsäure, um einen pH von etwa 8 bis 14 zu erhalten, Behandlung des pH-eingestellten Reaktionsgemisches mit 1,0 bis 4,0 molaren Äquivalenten Hypochlorit und Halten der Reaktionsmischung bei einer Temperatur zwischen etwa 25 bis 125 °C, um die Pyridin-2,3-dicarbonsäure der Formel I zu erhalten.

**2.** Verfahren gemäß Anspruch 1, ferner umfassend die Isolierung der Pyridin-2,3-dicarbonsäure der Formel I durch Ansäuern der Reaktionsmischung und Extraktion mit einem organischen Lösungsmittel.

**3.** Verfahren gemäß Anspruch 1, wobei etwa 7,5 bis 9,0 molare Äquivalente Wasserstoffperoxid und 5,0 bis 6,0 molare Äquivalente wässrige Base zu einer gerührten Lösung eines Chinolins der Formel II gegeben werden.

**4.** Verfahren gemäß Anspruch 3, wobei der pH-Bereich der Reaktionsmischung zur Zeit der Hypochloritzugabe 10,5 bis 11,5 beträgt.

**5.** Verfahren gemäß Anspruch 4, wobei 1,0 bis 2,0 molare Äquivalente Hypochlorit in situ erzeugt werden durch die Zugabe von Chlorgas.

**6.** Verfahren gemäß Anspruch 4, wobei 1,0 bis 2,0 molare Äquivalente Hypochlorit als eine 5 bis 15 %ige Lösung zugegeben werden.

**7.** Verfahren gemäß Anspruch 2, wobei das organische Lösungsmittel ausgewählt ist aus der Gruppe, bestehend aus Tetrahydrofuran, Aceton, $C_{2-6}$-Alkohol und Mono-$C_{1-4}$-Ethern von Ethylenglycol.

**8.** Verfahren gemäß Anspruch 7, wobei das organische Lösungsmittel Tetrahydrofuran ist.

## Revendications

**1.** Procédé de préparation d'acides pyridine-2,3-dicarboxyliques de formule I

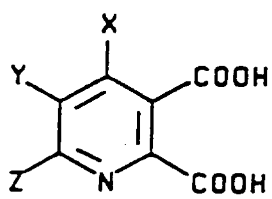

dans laquelle
X est un atome hydrogène ou un groupe méthyle, à condition que, lorsque Y et Z sont pris conjointement pour former un cycle et lorsque YZ est représenté par la structure : $-(CH_2)_n-$, dans laquelle n est égale à 3 ou 4, X soit un atome d'hydrogène ;
Y est un atome d'hydrogène, d'halogène, un groupe alkyle en $C_1$-$C_6$, hydroxy alkyle en $C_1$-$C_6$, halogénoalkyle en $C_1$-$C_6$, aminoalkyle en $C_1$-$C_6$, sulfonylakyle en $C_1$-$C_6$, nitro, hydroxy, formyle, carboxy, acyle, amido, amino, alkylamino en $C_1$-$C_4$, dialkylamino inférieur, alkylsulfonyle en $C_1$-$C_4$, sulfonamido ou phényl éventuellement substitué par un groupe alkyle en $C_1$-$C_4$, un groupe alkylsulfonyle en $C_1$-$C_4$, un atome d'halogène, un groupe hydroxy ou un groupe trifluorométhyle ;
Z est un atome d'hydrogène, un groupe alkyle en $C_1$-$C_6$, hydroxyalkyle en $C_1$-$C_6$, halogénoalkyle en $C_1$-$C_6$, aminoalkyle en $C_1$-$C_6$, sulfonylalkyle en $C_1$-$C_6$, nitro, hydroxy, formyle, carboxy, acyle, amido, amino, alkylamino en $C_1$-$C_4$, dialkylamino inférieur, alkylsulfonyle en $C_1$-$C_4$, sulfonamido, ou phényle éventuellement substitué par un groupe alkyle en $C_1$-$C_4$, un groupe alkylsulfonyle en $C_1$-$C_4$, un atome d'halogène, un groupe hydroxy ou un groupe trifluorométhyle ; et,
lorsqu'ils sont pris conjointement, Y et Z peuvent former un cycle dans lequel YZ sont représentés par la structure : $-(CH_2)_n-$, dans laquelle n est un nombre entier choisi parmi 3 ou 4, à condition que X soit

un atome d'hydrogène ; ou

$$\begin{matrix} L & M & Q & R_1 \\ & & & \end{matrix}$$
$$-C{=}C{-}C{=}C{-},$$

dans laquelle L, M, Q, et $R_1$ représentent chacun des radicaux choisis dans le groupe constitué par les groupes hydroxy, halogéno, alkyle en $C_1$-$C_4$, alkylsulfonyle en $C_1$-$C_4$, halogénoalkylamino en $C_1$-$C_4$, alkylamino en $C_1$-$C_4$, dialkylamino inférieur et trifluorométhyle, à condition que l'un seulement des radicaux L, M, Q ou $R_1$ puisse représenter un substituant autre qu'un atome d'hydrogène, un atome d'halogène ou un groupe alkyle en $C_1$-$C_4$,
comprenant la réaction d'un composé quinoléine de formule II

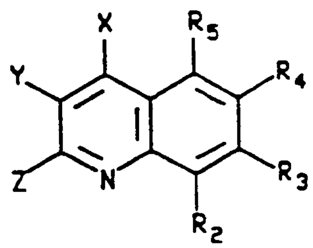

dans laquelle
X, Y et Z sont tels que décrits pour la formule I ci-dessus, $R_2$, $R_3$, $R_4$ et $R_5$ sont chacun des atomes d'hydrogène, des groupes hydroxy, $SO_3H$, $SO_2Cl$, SH, halogéno, $NO_2$, $NH_2$ ; à
condition que l'un des radicaux $R_2$, $R_3$, $R_4$ et $R_5$ soit différent d'un atome d'hydrogène et que, lorsque $R_2$ est un groupe hydroxy, l'un au moins des radicaux $R_3$, $R_4$ et $R_5$ soit différent d'un atome d'hydrogène ;
leurs N-oxydes ; leurs sels d'addition avec des acides ;
avec environ 7,0-20,0 équivalents molaires de peroxyde d'hydrogène, en présence de 2,0-10,0 équivalents molaires de base aqueuse, choisie par le groupe constitué par les hydroxydes et les carbonates de métaux alcalins et de métaux alcalino-terreux et leurs mélanges, dans un domaine de températures d'environ 25 °C-125 °C, le refroidissement du mélange réactionnel ainsi formé jusqu'à une température inférieure à 125 °C, le traitement du mélange réactionnel refroidi avec un acide minéral, pour obtenir un pH d'environ 8-14, le traitement du mélange réactionnel à pH ajusté avec 1,0-4,0 équivalents molaires d'hypochlorites et le maintien dudit mélange réactionnel à une température comprise entre 25 °C et 125 °C, pour donner l'acide pyridine-2,3-dicarboxylique de formule I.

**2.** Procédé selon la revendication 1, qui comprend, en outre, l'isolement de l'acide pyridine-2,3-dicarboxylique de formule I par acidification dudit mélange réactionnel et extraction avec un solvant organique.

**3.** Procédé selon la revendication 1, dans lequel on ajoute environ 7,5-9,0 équivalents molaires de peroxyde hydrogène et 5,0-6,0 équivalents molaires de base aqueuse à une solution agitée de quinoléine de formule II.

**4.** Procédé selon la revendication 3, dans lequel le domaine de pH du domaine réactionnel est de 10,5 à 11,5 au moment de l'addition de l'hypochlorite.

**5.** Procédé selon la revendication 4, dans lequel 1,0-2,0 équivalents molaires d'hypochlorite sont produits in situ par l'addition de chlore gazeux.

**6.** Procédé selon la revendication 4, dans lequel 1,0-2,0 équivalents molaires d'hypochlorite sont ajoutés sous forme d'une solution aqueuse à 5%-15%.

**7.** Procédé selon la revendication 2, dans lequel le solvant organique est choisi dans le groupe constitué par le tétrahydrofurane, l'acétone, un alcool en $C_3$-$C_6$ et des mono-éthers en $C_1$-$C_4$ d'éthylène glycol.

**8.** Procédé selon la revendication 7, dans lequel le solvant organique est le tétrahydrofurane.